(19) Europäisches Patentamt
European Patent Office
Office européen des brevets

(11) **EP 4 538 369 A1**

(12) **EUROPEAN PATENT APPLICATION**
published in accordance with Art. 153(4) EPC

(43) Date of publication:
**16.04.2025 Bulletin 2025/16**

(21) Application number: **22945944.1**

(22) Date of filing: **04.08.2022**

(51) International Patent Classification (IPC):
***C12N 9/26*** (2006.01)   ***A61K 38/47*** (2006.01)
***A61P 7/10*** (2006.01)

(52) Cooperative Patent Classification (CPC):
**A61K 38/47; A61P 7/10; C12N 9/2408**

(86) International application number:
**PCT/KR2022/011586**

(87) International publication number:
**WO 2023/238985 (14.12.2023 Gazette 2023/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.06.2022 KR 20220069769**

(71) Applicant: **BMI Korea Co. Ltd**
**Jeju-si, Jeju-do 63309 (KR)**

(72) Inventors:
• **WOO, Koo**
**Jeju-si, Jeju-do 63093 (KR)**
• **SHIN, Jaewook**
**Cheongju-si, Chungcheongbuk-do 28464 (KR)**
• **KIM, Nayoung**
**Seoul 08527 (KR)**

(74) Representative: **Grünecker Patent- und
Rechtsanwälte
PartG mbB
Leopoldstraße 4
80802 München (DE)**

Remarks:
The complete document including Reference
Table(s) and the Sequence Listing(s) can be
downloaded from the EPO website

(54) **HYALURONIDASE POLYPEPTIDE AND USE THEREOF**

(57) Disclosed herein are a hyaluronidase polypeptide and uses thereof.

**(Cont. next page)**

**FIG. 5b**

A) BMI2004 pH 4.0

B) BMI2004 pH 7.0

C) BMI2004 pH 10.0

**Description**

**TECHNICAL FIELD**

The present disclosure relates to a hyaluronidase polypeptide and a use thereof.

**BACKGROUND ART**

Hyaluronidases are a generic term for a family of enzymes that catalyze the degradation of hyaluronic acid. At first, Duran-Reynals introduced hyaluronidase as a spreading factor. Later, it has been called hyaluronidase as it was observed to exhibit potent activity on hyaluronic acid. According to the enzymatic mechanism thereof, the enzymes are classified into the following three types: hyaluronate 4-glycanohydrolases (EC 3.2.1.35) distributed in testicles, lysosomes, and bee venoms; hyaluronate 3-glycanohydrolases (EC 3.2.1.36) found in leaches; and hyaluronate lyases (EC 4.2.2.1) found in bacteria.

In particular, hyaluronidase (PH-20) in testicles is a glycosylphosphatidylinositol (GPI)-anchored enzyme present on the acrosomal part of a sperm and is an important enzyme that causes fertilization by degrading the thick outer wall of the egg. In addition, PH-20 is known to cleave the $\beta(1-4)$ linkage between D-glucuronic acid and N-acetyl-D-glucosamine found in hyaluronic acid, chondroitin, and chondroitin sulfate among glycosaminoglycans distributed in the mammalian skin. The general molecular formula of these enzymes is $C_{2455}H_{3775}N_{617}O_{704}S_{21}$ and a molecular weight is 53870.9 g/mol. In humans, there are six genes associated with the enzymes comprising HYAL1, HYAL2, HYAL3, and PH-20/SPAM1.

Since the 1950s, broad applications of hyaluronidases have been comprehensively reviewed. The first application was the subcutaneous injection of parenteral fluids. Besides, the enzymes have found applications in the fields of orthopedic, ophthalmology, plastic surgery, dental, oral surgery, gynecology, and otorhinolaryngology, such as use in infiltration and block anesthesia in order to increase the diffusion of local anesthetics and steroids, dispersion of collected body fluids, e.g., hematoma, prevention of peritoneal adhesion and calculus formation, and treatment of infertility.

Currently available on the market are hyaluronidases that are extracted from ovine or bovine testicles, as exemplified by Vitrase (ISTA Pharmaceuticals, ovine source), and Amphadase (Amphastar Pharmaceuticals, bovine source). They are commercialized by loading a suitable concentration of unprocessed hyaluronidases into vials, followed by lyophilization. The commercial products of animal-derived hyaluronidases leave many problems in application to various fields because foreign proteins contained therein may cause allergic responses and their biological activity decreases due to the decrease of stability with time.

To overcome such problems, research has been conducted on recombinant hyaluronidase. Recombinant proteins can be expressed in various types of cells comprising E. coli, yeasts, insect cells, animal cells, etc. Particularly as for hyaluronidases, their activities are affected by the glycosylation made in the posttranslational modification process. This is because glycans may have influences on the antigenicity, structural folding, solubility, and stability of glycoproteins. From this point of view, animal cells are suitable among various types of expression cells with the most preference for CHO (Chinese hamster ovary) cells which have secured safety, because yeasts or insect cells that glycosylation take place are different from mammalian cells in terms of the posttranslational modification process.

The first recombinant hyaluronidase of PH-20 was developed by Halozyme Therapeutic and has been sold under the tradename of Hylenex, and are being developed for various uses in subcutaneous injection, vitrectomy, and ocular disability, etc. However, hyaluronidases are still poor in yield or stability and are insufficiently supplied compared to demand, so there is a need for a hyaluronidase with improved yield or stability.

**DISCLOSURE**

**TECHNICAL PROBLEM**

An aspect of the present disclosure is to provide a hyaluronidase polypeptide with excellent stability and improved activity.

Another aspect of the present disclosure is to provide a composition for topical administration, comprising the polypeptide according to an aspect of the present disclosure.

A further aspect of the present disclosure is to provide a drug delivery carrier comprising the polypeptide according to an aspect of the present disclosure.

A still further aspect of the present disclosure is to provide a composition for prevention or treatment of edema, comprising the polypeptide according to an aspect of the present disclosure.

**TECHNICAL SOLUTION**

An aspect of the present disclosure pertains to a polypeptide in which a C-terminal region is deleted from the amino acid sequence of wild-type hyaluronidase.

Another aspect of the present disclosure pertains to a polypeptide including deletion of at least one consecutive amino acid of 1 to 203 amino acids from the C-terminus of the amino acid sequence of wild-type hyaluronidase.

A further aspect of the present disclosure pertains to a composition for topical administration, comprising the polypeptide.

A still further aspect of the present disclosure pertains to a drug delivery carrier comprising the polypeptide.

Still another aspect of the present disclosure pertains to a composition for prevention or treatment of edema, comprising the polypeptide.

Below, a detailed description will be given of the present disclosure.

An aspect of the present disclosure is drawn to a polypeptide having a sequence homology of 90% or higher with a polypeptide in which a C-terminal region is deleted from the amino acid sequence of wild-type hyaluronidase. The wild-type hyaluronidase may be a polypeptide consisting of the amino acid sequence of SEQ ID NO: 1.

The polypeptide according to an aspect of the present disclosure has hyaluronidase activity, and may have at least one of the following characteristics (1) to (6):

(1) stable at a pH 3 to 10, specifically, enzyme activity is 57% or higher of the initial activity after storage at a pH in the range between higher than 3 and 10 or less for 4 weeks, or enzyme activity is 32% or higher of the initial activity after storage at a pH of 3 to less than 5 for 4 weeks,

(2) stable at a temperature of -20 to 45°C, specifically, enzyme activity is 63% or higher of the initial activity after storage at a temperature less than 0°C for 4 weeks, enzyme activity is 83% or higher of the initial activity after storage at a temperature of 0 to 40°C for 4 weeks, or enzyme activity is 52% or higher of the initial activity after storage at a temperature of 40°C or higher for 4 weeks,

(3) high hyaluronidase activity, specifically, hyaluronidase activity is more than 1- to 3-fold compared to wild-type hyaluronidase,

(4) having a titer more than 1- to 3-fold of a titer of polypeptide consisting of the amino acid sequence of SEQ ID NO: 1,

(5) having an activity of 120,000 to 150,000 IU/mg, and

(6) one or more amino acid residue is glycosylated.

In detail, a polypeptide is prepared by deleting a C-terminal region of the amino acid sequence of ovine-derived wild-type hyaluronidase (CAS no.488712-31-8) in the present Example. The polypeptide thus obtained were observed to have stability and activity remarkably higher than those of the wild-type hyaluronidase, and even when used in a smaller amount, exhibited equivalent effects of drug absorption, drug diffusion promoting effects, and body fluid reabsorption effects to those of the wild-type. Therefore, the polypeptide according to an embodiment of the present disclosure may be a hyaluronidase.

In detail, the polypeptide according to an embodiment of the present disclosure may have a sequence homology of 90% or more, 91% or more, 92% or more, 93% or more, 94% or more, 95% or more, 96% or more, 97% or more, 98% or more, 99% or more, 99.5% or more, or 99.9% or more with a polypeptide comprising deletion of at least one consecutive amino acid of 1 to 203 amino acids from the C-terminus of the amino acid sequence of wild-type hyaluronidase. In this regard, the polypeptide according to an embodiment of the present disclosure is not the polypeptide consisting of the amino acid sequence of SEQ ID NO: 1. In addition, the polypeptide according to an embodiment of the present disclosure may retain the catalytic activity of the wild-type hyaluronidase. Specifically, the polypeptide according to an embodiment of the present disclosure may have activity and/or stability equal to or greater than that of wild-type hyaluronidase. The wild-type hyaluronidase may be a polypeptide consisting of the amino acid sequence of SEQ ID NO: 1.

Specifically, the polypeptide according to an embodiment of the present disclosure may be a C-terminal region deletion variant in which n amino acids from the C-terminus of the amino acid sequence of wild-type hyaluronidase is deleted (wherein, n is a natural number of 1 to 203).

By way of example, the polypeptide according to an embodiment of the present disclosure may have deletion of at least one consecutive amino acid of 1 to 203, 1 to 170, 1 to 136, 1 to 102, 1 to 68, 34 to 203, 34 to 170, 34 to 136, 34 to 102, 34 to 68, 68 to 203, 68 to 170, 68 to 136, 68 to 102, 34, 68, 102, 136, or 170 amino acids from the C-terminus of the amino acid sequence of wild-type hyaluronidase. The polypeptide according to an embodiment of the present disclosure may further have deletion of the first amino acid from the N-terminus of the amino acid sequence of wild-type hyaluronidase. The amino acid sequence of the wild-type hyaluronidase may be the amino acid sequence of SEQ ID NO: 1.

For one example, the polypeptide according to an embodiment of the present disclosure may consist of an amino acid sequence extending from the first amino acid to the $m^{th}$ amino acid or the second amino acid to the $m^{th}$ amino acid from the N-terminus of the amino acid sequence of wild-type hyaluronidase (wherein, m is a natural number of 315 to 517). The amino acid sequence of the wild-type hyaluronidase may be the amino acid sequence of SEQ ID NO: 1.

For one example, the polypeptide according to an embodiment of the present disclosure may consist of the amino acid sequence of SEQ ID NO: 2, SEQ ID NO: 3, SEQ ID NO: 4, SEQ ID NO: 5, or SEQ ID NO: 6.

For one example, the polypeptide according to an embodiment of the present disclosure may be expressed using animal cells as a host. In this regard, the polypeptide according to an embodiment of the present disclosure may be expressed in animal cells as a host, and glycosylated through post-translational modification (PTM) process when being expressed.

The polypeptide according to an embodiment of the present disclosure may be stable at a pH 3 to 10. Specifically, the polypeptide according to an embodiment of the present disclosure may decrease in enzymatic activity upon storage at a pH of 3 to 10 to a lesser extent, compared to the wild-type hyaluronidase.

In the Example of the present disclosure, the polypeptide according to an embodiment of the present disclosure was observed to retain the enzymatic activity after four weeks of storage at a pH of 3 to 10.

For example, the polypeptide according to an embodiment of the present disclosure may have an enzymatic activity amounting to 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 51% or more, 52% or more, 53% or more, 54% or more, or 55% or more of the initial activity thereof after storage at a pH in the range between pH 3 and less than pH 5, or pH 3 to 4, e.g., pH 3 for 4 weeks. In this context, the polypeptide may be stored at a temperature of 5°C or 37°C.

For example, the polypeptide according to an embodiment of the present disclosure may have an enzymatic activity amounting to 57% or more, 58% or more, 59% or more, 60% or more, 61% or more, 62% or more, 63% or more, 64% or more, or 65% or more of the initial activity thereof after storage at a pH in the range between higher than pH 3 and pH 10 or less, pH 4 to 10, or pH 5 to 10, for example, pH 5, pH 7, or pH 10 for 4 weeks. In this context, the polypeptide may be stored at a temperature of 5°C or 37°C.

For example, the polypeptide according to an embodiment of the present disclosure may have an enzymatic activity of 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, or 82% or more of the initial activity thereof after storage at a pH in the range between higher than pH 3 and less than pH 10, between higher than pH 3 and pH 9 or less, between higher than pH 3 and pH 8 or less, between higher than pH 3 and pH 7 or less, between pH 4 or more and less than pH 10, pH 4 to 9, pH 4 to 8, pH 4 to 7, between pH 5 and less than pH 10, pH 5 to 9, pH 5 to 8, or pH 5 to 7, e.g., pH 5 or pH 7 for 4 weeks. In this context, the polypeptide may be stored at a temperature of 5°C or 37°C.

The polypeptide according to an embodiment of the present disclosure may be stable in a frozen, a refrigerated, and a high-temperature condition. In detail, when stored in a frozen, a refrigerated, and a high-temperature condition, the polypeptide according to an embodiment of the present disclosure decreases in enzymatic activity to a lesser extent, compared to the wild-type hyaluronidase.

In the present Example, the polypeptide according to an embodiment of the present disclosure was observed to retain an enzymatic activity after 4 weeks of storage in a frozen (e.g., -18°C to -20°C), a refrigerated (e.g., 2 to 8°C), and a high-temperature (e.g., 40 to 45°C) condition.

For example, the polypeptide according to an embodiment of the present disclosure may have an enzymatic activity amounting to 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more of the initial activity after storage at a frozen temperature, for example, a temperature of less than 0°C, -20 to less than 0°C, -20 to -10°C, or -20 to - 18°C, for one example, at a temperature of -20°C, for 4 weeks. In this regard, the polypeptide may be stored at pH 5 or 7.

For example, the polypeptide according to an embodiment of the present disclosure may have an enzymatic activity amounting to 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more of the initial activity after storage at a refrigerated temperature, for example, a temperature of 0 to 40°C, 0 to 10°C, or 2 to 8°C, e.g., 5°C for 4 weeks. In this regard, the polypeptide may be stored at pH 5 or 7.

For example, the polypeptide according to an embodiment of the present disclosure may have an enzymatic activity amounting to 52% or more, 53% or more, 54% or more, 55% or more, 56% or more, 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, or 79% or more of the initial activity after storage at a high temperature, for example, a temperature of 40°C or more, 40 to 50°C, or 40 to 45°C, e.g., 40°C for 4 weeks. In this regard, the polypeptide may be stored at a pH of 5 or 7.

The polypeptide according to an embodiment of the present disclosure may have a titer higher than that of the wild-type hyaluronidase. For example, the polypeptide according to an embodiment of the present disclosure may have a titer more than 1- to 3-fold, more than 1- to 2.5-fold, more than 1- to 2-fold, more than 1- to 1.9-fold, more than 1- to 1.8-fold, more than 1- to 1.7-fold, 1.1- to 3-fold, 1.1- to 2.5-fold, 1.1- to 2-fold, 1.1- to 1.9-fold, 1.1- to 1.8-fold, 1.1- to 1.7-fold, 1.2- to 3-fold , 1.2- to 2.5-fold , 1.2-to 2-fold, 1.2- to 1.9-fold, 1.2- to 1.8-fold, 1.2- to 1.7-fold, 1.3- to 3-fold, 1.3- to 2.5-fold, 1.3- to 2-fold, 1.3- to 1.9-fold, 1.3- to 1.8-fold, 1.3- to 1.7-fold, 1.4- to 3-fold, 1.4- to 2.5-fold, 1.4- to 2-fold, 1.4- to 1.9-fold, 1.4- to 1.8-fold, 1.4- to 1.7-fold, 1.5- to 3-fold, 1.5- to 2.5-fold, 1.5- to 2-fold, 1.5- to 1.9-fold, 1.5- to 1.8-fold, or 1.5- to 1.7-fold of a titer of the wild-type hyaluronidase.

For example, the polypeptide according to an embodiment of the present disclosure may have an activity of 120,000 to 150,000 IU/mg, 120,000 to 145,000 IU/mg, 120,000 to 140,000 IU/mg, 120,000 to 135,000 IU/mg, 121,000 to 150,000 IU/mg, 121,000 to 145,000 IU/mg, 121,000 to 140,000 IU/mg, 121,000 to 135,000 IU/mg, 122,000 to 150,000 IU/mg, 122,000 to 145,000 IU/mg, 122,000 to 140,000 IU/mg, or 122,000 to 135,000 IU/mg.

Another aspect of the present disclosure is drawn to a nucleic acid molecule encoding the polypeptide according to an embodiment of the present disclosure, a vector comprising the nucleic acid molecule, and a cell comprising the vector. The cell may be selected from the group consisting of bacteria comprising E. coli and Actinomyces, yeasts, fungi, insect cells, animal cells, mammalian cells, algal cells, and plant cells. The mammalian cells may be selected from the group consisting of CHO, NS0, HEK293, BHK, Per.C6, MDCK, Vero, MRC, HeLa, IMR, and Sp2/0. The CHO cells may be selected from the group consisting of CHO-DG44, CHO-DUKX, CHO-S, CHO-K1, and CHO-DP12.

A further aspect of the present disclosure is drawn to a composition for topical administration, comprising the polypeptide according to an embodiment of the present disclosure. The composition may be adapted for subcutaneous administration. In the Example of the present disclosure, when topically administered, the polypeptide according to an embodiment of the present disclosure was observed to exhibit remarkably better effects of drug absorption and diffusion promoting, compared to the wild-type hyaluronidase. Thus, another embodiment of the present disclosure is drawn to a drug delivery carrier comprising the polypeptide according to an embodiment of the present disclosure.

In addition, in the present Example, when topically administered, the polypeptide according to an embodiment of the present disclosure was observed to exhibit a remarkably higher effect of promoting reabsorption of excessive body fluid, compared to the wild-type hyaluronidase. Thus, another embodiment of the present disclosure is drawn to a pharmaceutical composition for prevention or treatment of edema, comprising the polypeptide according to an embodiment of the present disclosure as an active ingredient

The composition of the present disclosure, e.g., the pharmaceutical composition may further comprise at least one active ingredient that exhibits an equivalent or similar function.

In addition, according to the conventional techniques known to those skilled in the art, the composition of the present disclosure, for example, a pharmaceutical composition, may be formulated with a pharmaceutically acceptable carrier and prepared into a single-dose form or enclosed in a multiple-dose container. As used herein, the term "carrier" refers to a substance that facilitates the incorporation of a matter of interest into cells or tissues. Herein, the term "pharmaceutically acceptable" means pertaining to being physiologically acceptable and not causing gastrointestinal disorders, allergic reactions such as dizziness, or similar reactions when administered to a human.

Any pharmaceutical acceptable carrier may be used so long as it is typically available for formulation. Examples of the pharmaceutical acceptable carrier comprise, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil.

In addition to the above ingredient, the composition according to the present disclosure, for example, a pharmaceutical composition may further comprise an additive such as a filler, an antiaggregant, a lubricant, a humectant, a flavor, an emulsifier, a preservative, etc. In the present disclosure, the content of the additive in the composition is not particularly limited, but may be appropriately adjusted within the content range acceptable for typical formulation.

The term "excipient", as used herein, means any substance, not itself a therapeutic agent, used as a carrier or vehicle for delivery of a therapeutic agent to a subject or added to a pharmaceutical composition to improve its handling or storage properties or to permit and facilitate of a unit dose of the composition.

The composition according to the present disclosure, for example, a pharmaceutical composition, may be formulated into various forms, such as sterile injections, etc., according to purposes and may be administered via various routes, such as topical, subcutaneous, intramuscular injection, etc.

A preferable dosage of the composition according to the present disclosure, for example, a pharmaceutical composition, may vary in a range thereof depending on a patient's state, weight, age, sex, health condition, dietary constitutional specificity, property of a preparation, a degree of disease, an administration time of the composition, an administration method, an administration period or interval, an excretion rate, and drug form, but may be appropriately selected by those skilled in the art.

The term "effective dosage of the pharmaceutical composition", as used herein, means an amount of the composition comprising an active ingredient sufficient to treat a specific symptom. This may vary depending on a method for formulating the pharmaceutical composition, an administration mode, an administration time and/or an administration route, etc., and may be diversified according to various factors comprising a type and degree of reaction to be achieved by administration of the pharmaceutical composition, a type of an individual for administration, the individual's age, weight, general health condition, disease symptom or severity, sex, diet, excretion, ingredients of other drug compositions to be used for the corresponding individual at the same time or different times, etc., as well as other similar factors well known in a pharmaceutical field, and those skilled in the art may easily determine and prescribe an effective dosage for intended treatment.

The pharmaceutical composition according to the present disclosure may be administered once a day or divided into several times. The pharmaceutical composition of the present invention may be administered as an individual therapeutic agent or in combination with other therapeutic agents, and may be administered sequentially or simultaneously with a conventional therapeutic agent. Considering all the above factors, the pharmaceutical composition of the present disclosure may be administered in such an amount that a maximum effect may be achieved by a minimum amount without a side effect.

Compared to the wild-type hyaluronidase, the polypeptide or the composition according to an embodiment of the present disclosure has a higher titer and thus can achieve an equivalent effect at a lower dose. For example, the polypeptide or the composition according to an embodiment of the present disclosure may be administered to at a daily dose of 90% or less, 85% or less, 80% or less, 75% or less, 70% or less, or 65% or less of that of the wild-type hyaluronidase. The dose may be based on weight or % by weight. In addition, a total daily dose may be divided and administered continuously or discontinuously as necessary.

## ADVANTAGEIOUS EFFECTS

With an improvement in expression level and stability in animal cells, the hyaluronidase polypeptide according to an embodiment of the present disclosure exhibits enzymatic activity equal to or greater than the mature wild-type PH-20. Therefore, the hyaluronidase polypeptide according to an embodiment of the present disclosure has an elevated expression level when expressed in CHO cells compared to the mature wild-type PH-20, and is highly stable, there is an effect of increasing the industrial applicability of various uses.

In addition, with ability to degrade hyaluronic acid as a constituent of the intercellular space, hyaluronidases adjust tissue adhesion to facilitate the penetration and diffusion of drugs, and have the effect of promoting reabsorption of body fluids excessively present in tissues, so that their coverage and application fields have been gradually expanded. However, animal-derived wild-type PH-20 is highly prone to infection with the substance originating from the animal source. The hyaluronidase polypeptide according to an embodiment of the present disclosure is unlikely to cause infection and thus safe, the peptide exhibits high activity at the same dose compared to animal-derived wild-type PH-20, and can further increase industrial applicability for various uses.

## DESCRIPTION OF DRAWINGS

FIG. 1a to FIG. 1c are drawings of SDS-PAGE showing the pH stability of a polypeptide according to an embodiment of the present disclosure in comparison with wild-type hyaluronidase (FIG. 1a: week 0 of storage, FIG. 1b: week 2 of storage, FIG. 1c: week 4 of storage).

FIG. 2a to FIG. 2c are drawings of SDS-PAGE showing the temperature stability of a polypeptide according to an embodiment of the present disclosure in comparison with wild-type hyaluronidase (FIG. 2a: week 0 of storage, FIG. 2b: week 2 of storage, FIG. 2c: week 4 of storage; frozen: frozen, refrig.: refrigerated, hig tem.: high-temperature).

FIG. 3 shows the results of plate titer assays under various pH and temperature conditions to examine whether a hyaluronic acid solution exhibits its own activity.

FIG. 4a shows the results of titer assays for wild-type hyaluronidase under the temperature condition of 20 to 60°C.

FIG. 4b shows the results of titer assays for a polypeptide according to an embodiment of the present disclosure under the temperature condition of 20 to 60°C.

FIG. 5a shows the results of titer assays for wild-type hyaluronidase under the temperature condition of 35 to 40°C.

FIG. 5b shows the results of titer assays for a polypeptide according to an embodiment of the present disclosure under the temperature condition of 35 to 40°C.

FIG. 6a shows the results of titer assays for wild-type hyaluronidase under the condition of pH 5 to 7.

FIG. 6b shows the results of titer assays for a polypeptide according to an embodiment of the present disclosure under the condition of pH 5 to 7.

FIG. 7 shows plots of blood levels of a polypeptide according to an embodiment of the present disclosure and Hirax against time in SD rats.

## MODE FOR INVENTION

A better understanding of the present disclosure may be obtained in light of following examples which are set forth to illustrate, but are not to be construed to limit, the present disclosure.

### EXAMPLE 1. Preparation of Hyaluronidase

A hyaluronidase was prepared by truncating regions extending from the C-terminus of the amino acid sequence of SEQ ID NO: 1 for the ovine-derived wild-type hyaluronidase (CAS no.488712-31-8) by 34 amino acid residues (Example 1-2), 68 amino acid residues (Example 1-3), 102 amino acid residues (Example 1-4), 136 amino acid residues (Example 1-5), 170 amino acid residues (Example 1-6), or 204 amino acid residues (Example 1-7).

First, a cDNA was synthesized on the basis of the amino acid sequence of wild-type hyaluronidase (CAS no.488712-31-8). The gene of the hyaluronidase was amplified using polymerase chain reaction (hereinafter referred to as "PCR") and examined for expression and activity in CHO-DG44 cells with the aid of pcDNA 3.1 vector. The gene was anchored in CHO-DG44 cells through a pOptiVEC vector. When CHO-DG44 cells reached to a density of 4~6 x $10^6$ cells/mL, the cells were transformed with a plasmid constructed by inserting the hyaluronidase cDNA into pOptiVEC vector, using electroporation. After transformation, the CHO-DG44 cells were cultured in Power CHO 2CD medium (with L-glutamine 4mM) and the cell culture was centrifuged at 12,000 rpm for 10 minutes. The supernatant was recovered and then purified, as necessary, by various methods including affinity chromatography, hydrophobic chromatography, ion exchange chromatography, and the like. The purified hyaluronidase was filtered by replacement with water in ultrafiltration and microfiltration. Amino acid sequences of the hyaluronidases thus obtained are given in Table 1.

TABLE 1

| name | Sequence (N → C) | SEQ ID NO: |
|---|---|---|
| Example 1-1 (CAS no.488712-31-8) | LDFRAPPLIS NTSFLWAWNA PAERCVKIFK LPPDLRLFSV KGSPQKSATG QFITLFYADR LGYYPHIDEK TGNTVYGGIP QLGNLKNHLE KAKKDIAYYI PNDSVGLAVI DWENWRPTWA RNWKPKDVYR DESVELVLQK NPQLSFPEAS KIAKVDFETA GKSFMQETLK LGKLLRPNHL WGYYLFPDCY NHNYNQPTYN GNCSDLEKRR NDDLDWLWKE STALFPSVYL NIKLKSTPKA AFYVRNRVQE AIRLSKIASV ESPLPVFVYH RPVFTDGSST | 1 |
| | YLSQGDLVNS VGEIVALGAS GIIMWGSLNL SLTMQSCMNL GNYLNTTLNP YIINVTLAAK MCSQVLCHDE GVCTRKQWNS SDYLHLNPMN FAIQTGKGGK YTVPGKVTLE DLQTFSDKFY CSCYANINCK KRVDIKNVHS VNVCMAEDIC IEGPVKLQPS DHSSSQNEAS TTTVSSISPS TTATTVSPCT PEKQSPECLK VRCLEAIANV TQTGCQGVKW KNTSSQSSIQ NIKNQTTY | |

(continued)

| name | Sequence (N → C) | SEQ ID NO: |
|---|---|---|
| Example 1-2 | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTMQSCMNLG NYLNTTLNPY IINVTLAAKM CSQVLCHDEG VCTRKQWNSS DYLHLNPMNF AIQTGKGGKY TVPGKVTLED LQTFSDKFYC SCYANINCKK RVDIKNVHSV NVCMAEDICI EGPVKLQPSD HSSSQNEAST TTVSSISPST TATTVSPCTP EKQSPECLKV RCL | 2 |
| Example 1-3 (BMI2004) | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTMQSCMNLG NYLNTTLNPY IINVTLAAKM CSQVLCHDEG VCTRKQWNSS DYLHLNPMNF AIQTGKGGKY TVPGKVTLED LQTFSDKFYC SCYANINCKK RVDIKNVHSV NVCMAEDICI EGPVKLQPSD HSSSQNEAS | 3 |
| Example 1-4 | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTMQSCMNLG NYLNTTLNPY IINVTLAAKM CSQVLCHDEG VCTRKQWNSS DYLHLNPMNF AIQTGKGGKY TVPGKVTLED LQTFSDKFYC SCYANINCKK RVDIK | 4 |
| Example 1-5 | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG | 5 |

(continued)

| name | Sequence (N → C) | SEQ ID NO: |
|---|---|---|
| | KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTMQSCMNLG NYLNTTLNPY IINVTLAAKM CSQVLCHDEG VCTRKQWNSS DYLHLNPMNF AIQTGKGGKY T | |
| Example 1-6 | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTMQSCMNLG NYLNTTLNPY IINVTLAAKM CSQVLCH | 6 |
| Example 1-7 | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTM | 7 |

**EXAMPLE 2. Assay for Stability of Hyaluronidases According to Deletion Site**

**(1) Comparison of enzymatic activity or content by storage pH according to deletion site**

Among the hyaluronidases prepared in Example 1, six hyaluronidases, except for the hyaluronidase of Example 1-7 which lacks activity, were assayed for pH stability according to deletion site. Each amino acid sequence was expressed in transient cells and the cell cultures thus obtained were concentrated in the same manner before measuring enzymatic activity. The experiment concentration was adjusted into 1,500 IU/mL (=100%), solutions were prepared according to the conditions shown in Table 2 by substituting with water, and stored at 37°C for 4 weeks under the condition of pH 3.0, pH 5.0, pH 7.0, or pH 10.0 while titration was conducted every two weeks.

The specimens with different deletion sites were assayed for enzymatic activity or content at each time point according to the following protocols:

1) The activity of hyaluronidases with different deletion sites was determined by proportion comparison with EP STD.
1-1) Preparation of pH 6.4 phosphate buffered saline: a solution of 2.5 g of disodium hydrogen phosphate dodecahydrate, 2.5 g of sodium phosphate dihydrogen, and 8.2 g of sodium chloride in 950 mL of water was adjusted with 1 M sodium hydroxide or 1 M hydrochloride to have a pH of 6.4 and added with water to form a final volume of 1,000 mL.
1-2) Preparation of dilution: In a mixture of 100 mL of pH 6.4 phosphate-buffered saline and 100 mL of water, 0.140 g of a gelatin reagent was dissolved at 37°C. The dilution should be used within 2 hours after preparation.
1-3) Preparation of substrate solution: To 0.5 g of sodium hyaluronate, 100 mL of water was added little by little while stirring. Water was slowly added until sodium hyaluronate swelled. At 4°C, stirring was continued for 12 hours or longer. The substrate solution thus obtained was stored at 4°C and should be used within 4 days after preparation.

1-4) Preparation of standard solution: EP STD (EDQM) reference standard was dissolved in the dilution to give a concentration of about 50 IU/mL. Exact 3 mL of this solution was taken and put in a 250 mL volumetric flask to which the dilution was then added to form an exact volume of 250 mL. The resulting solution was used as a standard solution.

1-5) Preparation of test solution: The hyaluronidase test solutions with different deletion sites, each having about 0.6 IU/mL, were adjusted into the predetermined pH values and then diluted before use.

2) Manual: The standard and test solutions were tested according to the following method.

2-1) In a water bath maintained at 37°C, a 50-mL conical tube where 7.5 mL of pH 6.4 phosphate-buffered saline and 5.0 mL of the substrate solution had been mixed was left to stand.

2-2) To the conical tube containing pH 6.4 phosphate-buffered saline and the substrate solution, 2.5 mL of a test solution was added and mixed for 1 minute.

2-3) To a Ubbelohde microviscometer (DIN 51 562, Part 2, capillary type MIII, constant: about 0.1 $mm^2/s^2$ or an equivalent viscometer), all of the mixed solution in the conical tube was loaded.

2-4) The time taken for the solution to flow down from the upper indication line to the lower indication line in the Ubbelohde microviscometer was recorded using a second chronometer.

2-5) The time was recorded several times during about 20 minutes.

2-6) The above procedure was repeated three times.

3) Calculation: Titers (IU/mg) were calculated according to the equation.

3-1) Reaction time: $T_1 + T_2/2$

3-2)

$$\eta r^{-1} : \{(k \times T_2) / 0.6915\}^{-1}$$

$T_1$ : time taken for the solution to ascend to the upper indication line in the Ubbelohde microviscometer (second)

$T_2$ : $T - T_1$

$T$ : time taken for the solution to descend to the lower indication line in the Ubbelohde microviscometer (second)

$k$ : Ubbelohde microviscometer constant ($mm^2/s^2$), refer to the specification of Ubbelohde microviscometer

0.6915: the kinematic viscosity of the substrate solution at 37°C ($mm^2/s^2$)

3-3) Activity calculation: $(B_T/B_R)*(E_R E_T)*A$

$B_T$ : slopes of the regression curves plotted for test solutions in a coordinate with reaction time on the x axis and natural log $\eta r^{-1}$ on the y axis

$B_R$ : slope of the regression curve plotted for standard solution in a coordinate with reaction time on the x axis and natural log $\eta r^{-1}$ on the y axis

$E_T$ : concentration of test solution (mg/mL)

$E_R$ : concentration of standard solution (mg/mL)

$A$ : titer of standard solution (IU/mg)

TABLE 2

| Specimen | Activity | Amino acid at cleaved site | Proportion of deleted enzyme region (%) | Storage pH | Storage Temp. |
|---|---|---|---|---|---|
| Example 1-1 | Hyaluronidase (Transient Cell) | L-Y | about 0% | pH 7.0 | 37 °C |
| Example 1-2 | | D-L | about 6% | | |
| Example 1-3 | | D-S | about 12% | | |
| Example 1-4 | | D-K | about 18% | | |
| Example 1-5 | | D-T | about 24% | | |
| Example 1-6 | | D-H | about 30% | | |
| Example 1-7 | | D-M | about 36% | | |

Test data for pH stability are shown in Table 3, below. The enzyme activity (in IU/mL) in each test is expressed as percentages of the reference value 1,500 IU/mL (=100%), which is the initial enzyme activity at week 0. The enzyme activity ratio was calculated according to the following equation:

$$\text{Enzyme Activity Ratio (\%)} = \text{(enzyme activity at measured time)/(initial enzyme activity)} * 100$$

TABLE 3

| Enzyme Activity Ratio (%) | pH3.0 | | | pH 5.0 | | | pH 7.0 | | | pH 10.0 | | |
|---|---|---|---|---|---|---|---|---|---|---|---|---|
| Specimen | Week 0 | Week 2 | Week 4 | Week 0 | Week 2 | Week 4 | Week 0 | Week 2 | Week 4 | Week 0 | Week 2 | Week 4 |
| Example 1-1 | 100.47 | 61.31 | 31.62 | 100.43 | 72.84 | 55.30 | 100.36 | 73.17 | 56.03 | 100.88 | 63.31 | 45.92 |
| Example 1-2 | 102.00 | 84.77 | 55.05 | 101.17 | 94.52 | 80.57 | 101.47 | 95.88 | 81.24 | 101.25 | 86.29 | 64.98 |
| Example 1-3 | 102.66 | 85.48 | 55.26 | 101.80 | 95.02 | 81.19 | 101.49 | 96.22 | 82.55 | 102.68 | 89.95 | 65.11 |
| Example 1-4 | 101.23 | 84.91 | 54.76 | 101.19 | 94.96 | 80.65 | 101.38 | 95.50 | 81.73 | 101.49 | 88.27 | 64.19 |
| Example 1-5 | 100.46 | 83.59 | 52.94 | 100.15 | 94.88 | 77.45 | 100.60 | 93.87 | 78.81 | 100.67 | 86.74 | 62.64 |
| Example 1-6 | 100.82 | 82.32 | 50.58 | 100.08 | 92.61 | 74.88 | 99.32 | 91.36 | 77.63 | 99.75 | 85.76 | 61.17 |

As shown in Table 3, the pH stability data obtained for hyaluronidases with different deletion sites for 4 weeks indicates higher enzyme activity ratios (%) in the pH range of 5.0 to 7.0 than the other pH values. The hyaluronidases of Examples 1-2 to 1-6 exhibited remarkably high pH stability compared to the wild-type hyaluronidase of Example 1-1, with superiority of the hyaluronidases of Examples 1-2 to 1-4 to the other hyaluronidases. Values exceeding 100% seemed to be measurement errors that generally occurs due to large fluctuations in the titer tests of biological products such as vaccines and recombinant proteins (cytokines, monoclonal antibodies, etc.).

**(2) Comparison of enzyme activity or content by storage temperature according to deletion site**

The hyaluronidases of Examples 1-1 to 1-6 were assayed for temperature stability in the same manner as in (1) of Example 2. Solutions were prepared according to the conditions listed in Table 2 and stored at a frozen temperature (-20°C), a refrigerated temperature (5°C), and a high temperature (40°C) for 4 weeks during which a titer test was conducted every two weeks. The hyaluronidases with different truncated sites were measured for stability against temperature for 4 weeks and the measurements are shown in Table 4.

TABLE 4

| Enzyme Activity Ratio (%) | Frozen | | | Refrigerated | | | High temperature | | |
|---|---|---|---|---|---|---|---|---|---|
| Specimen | Week 0 | Week 2 | Week 4 | Week 0 | Week 2 | Week 4 | Week 0 | Week 2 | Week 4 |
| Example 1-1 | 100.22 | 81.47 | 62.51 | 100.54 | 90.89 | 82.55 | 98.13 | 65.09 | 51.55 |
| Example 1-2 | 101.98 | 100.74 | 98.85 | 101.52 | 100.81 | 99.45 | 98.96 | 88.92 | 78.54 |
| Example 1-3 | 102.68 | 100.88 | 99.22 | 102.77 | 101.91 | 100.23 | 99.47 | 89.90 | 79.63 |
| Example 1-4 | 101.07 | 100.17 | 98.03 | 100.32 | 100.72 | 99.18 | 98.16 | 88.11 | 77.95 |
| Example 1-5 | 100.12 | 98.82 | 95.52 | 100.53 | 98.70 | 97.51 | 99.39 | 85.31 | 75.78 |
| Example 1-6 | 99.49 | 96.92 | 93.12 | 99.64 | 96.32 | 95.63 | 99.87 | 82.69 | 73.12 |

As shown in Table 4, the hyaluronidases of Examples 1-2 to 1-6 exhibited great temperature stability and retained remarkably high enzyme activity ratios (%) particularly in the frozen and refrigerated storage conditions. The amino acid D-S hyaluronidase corresponding to Example 1-3 which was truncated by about 12%, showing the highest activity among the recombinant hyaluronidases, was named BMI2004 and used in the experiments.

### EXAMPLE 3. Assay for Stability of Wild-Type Hyaluronidase and BMI2004

### (1) Comparison of enzyme activity or content according to pH

Comparison was made of pH stability between BMI2004 prepared in Example 1 and a wild-type hyaluronidase. The BMI2004 and wild-type hyaluronidase (manufacturer; Korea BMI, tradename; Hirax, hereinafter referred to as Hirax) used in the pH stability test were 95% or higher in purity and each had a concentration of 1,500 IU/mL (=100%), solutions were prepared according to the conditions in Table 5 by substituting with water, and stored at 5°C under the conditions of pH 3.0, pH 5.0, pH 7.0, and pH 10.0 for 4 weeks during which a titer test was conducted every two weeks. At each time point, the specimens were measured for enzyme activity or content in the same manner as in (1) of Example 2.

TABLE 5

| pH | Specimen | Storage Temp. |
|---|---|---|
| 3.0 | BMI2004 / Hirax (1,500 IU/mL) | 2-8°C |
| 5.0 | | 2-8°C |
| 7.0 | | 2-8°C |
| 10.0 | | 2-8°C |

Measurements of the pH stability assay are shown in Table 6. The enzyme activity (in IU/mL) in each test is expressed as percentages of the reference value 1,500 IU/mL (=100%), which is the initial enzyme activity at week 0. The enzyme activity ratio was calculated according to the following equation:

$$\text{Enzyme Activity Ratio (\%)} = (\text{enzyme activity at measured time})/(\text{initial enzyme activity}) * 100$$

TABLE 6

| Enzyme Activity Ratio (%) | pH3.0 | | | pH 5.0 | | | pH 7.0 | | | pH 10.0 | | |
|---|---|---|---|---|---|---|---|---|---|---|---|---|
| Specimen | Week 0 | Week 2 | Week 4 | Week 0 | Week 2 | Week 4 | Week 0 | Week 2 | Week 4 | Week 0 | Week 2 | Week 4 |
| BMI2004 | 102.66 | 85.48 | 55.26 | 101.80 | 95.02 | 81.19 | 101.49 | 96.22 | 82.55 | 102.68 | 89.95 | 65.11 |
| Hirax | 102.04 | 51.11 | 31.03 | 103.39 | 71.20 | 50.40 | 102.33 | 70.76 | 50.95 | 101.88 | 62.07 | 41.03 |

As shown in Table 6, stability of BMI2004 and Hirax according to pH were observed during about 4 weeks. Higher enzyme activity ratios (%) were measured at pH 5.0 and pH 7.0 than pH 3.0 and pH 10.0. After week 2, the content of Hirax (%) tended to rapidly decrease, compared to BMI2004. Values exceeding 100% seemed to be measurement errors that generally occurs due to large fluctuations in the titer tests of biological products such as vaccines and recombinant proteins (cytokines, monoclonal antibodies, etc.). In order to additionally test the change in enzyme activity (or content) for stability confirmation according to pH of Hirax and BMI2004, SDS-PAGE (Sodium Dodecyl Sulfate-PolyAcrylamide Gel Electrophoresis) was performed under the conditions of Table 5 above, and according to the following protocol:

1) Preparation of sample buffer (5X): Pierce™ Lane Marker Reducing Sample Buffer (Thermo Scientific™, Cat No. 39000) was used.
2) Preparation of running buffer (1X): Novex™ Tris-Glycine SDS Running Buffer (10X) (Invitrogen, Cat No. LC2675) was used. Exact 100 mL of the solution was taken and put in a 1,000-mL volumetric flask to which water was then added to form an exact volume of 1,000 mL.

3) Preparation of test solution: reference standards for Hirax and BMI2004 were modified with water according to the conditions. About 20 μL of each of the solutions and 5 μL of the sample buffer (5X) were exactly taken and mixed in an EP tube.

4) Manual: Test solutions and PageRuler Prestained Protein Ladder reference standard (Thermo, Cat No. 26616) were subjected to the following assays.

4-1) After removal of a comb therefrom, Novex™ Wedgewell™ 8~16% Tris-Glycine Gel (Invitrogen, Cat No. XP08160BOX) or a sheet of equivalent gel in was cleansed with water to substitute for the buffer.

4-2) The washed gel was installed into the Mini Gel Tank. In the Mini Gel Tank, the cathode compartment was fully filled with running buffer (1X) while the anode compartment was filled about 2/3 with the running buffer (1X).

4-3) Into the gel, 7 μL of PageRuler Prestained Protein Ladder standard and 25 μL of the test solution were loaded.

4-4) The Mini Gel Tank was connected to a power supply and manipulated as follows, after which the specimens were run to 90% of the gel length:

Volt.: 140 V

Ampere: 400 mA

Time: 60 minutes (the time may be changed depending on the running condition.)

4-5) After completion of the running, the gel was separated from the cast and washed with water.

4-6) The washed gel was immersed in a tray containing a staining reagent (Coomassie Brilliant Blue R-250 Staining Solution, BIO-RAD, Cat No. 1610436).

4-7) The tray was put on a Rocker and shaken at 30 rpm for 30 minutes.

4-8) After completion of the staining, the gel was transferred to a tray containing a destaining solution (Coomassie Brilliant Blue R-250 Destaining Solution, BIO-RAD, Cat No. 1610438) and the tray was put on a rocker and shaken at 30 rpm until the gel was destained while the destaining solution was changed with a fresh one. When the destaining was conducted to some extent, the gel was immersed in water to remove the destaining solution.

4-9) When the destaining solution was removed therefrom, the gel was observed on a white lamp.

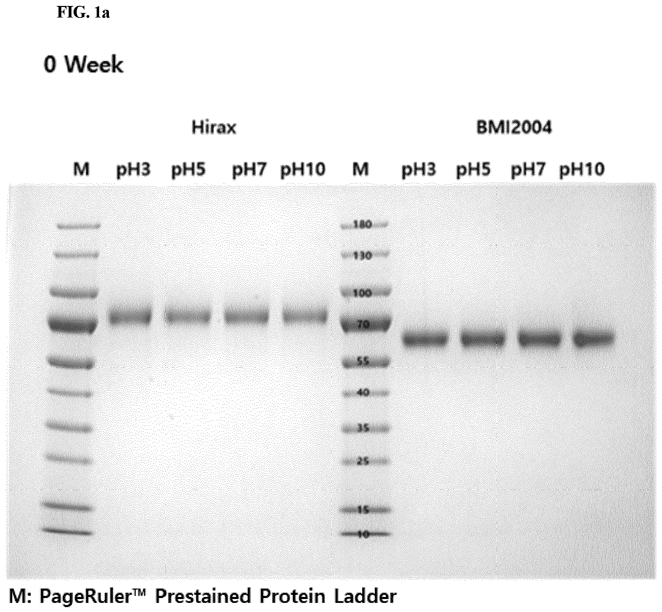

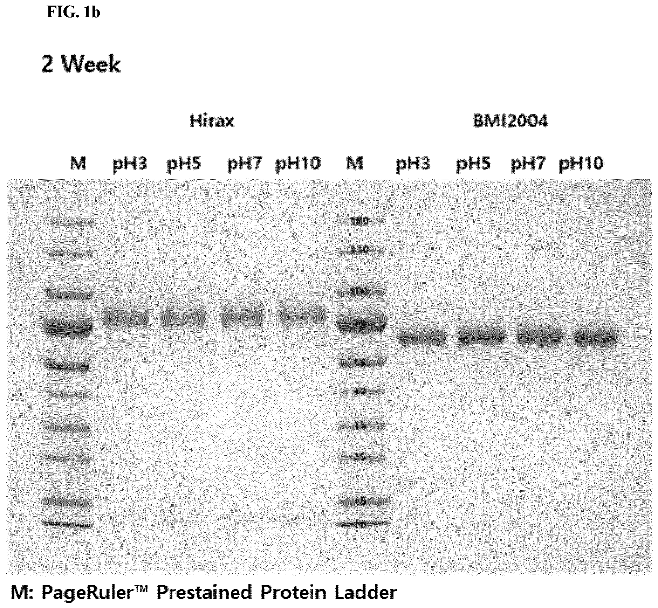

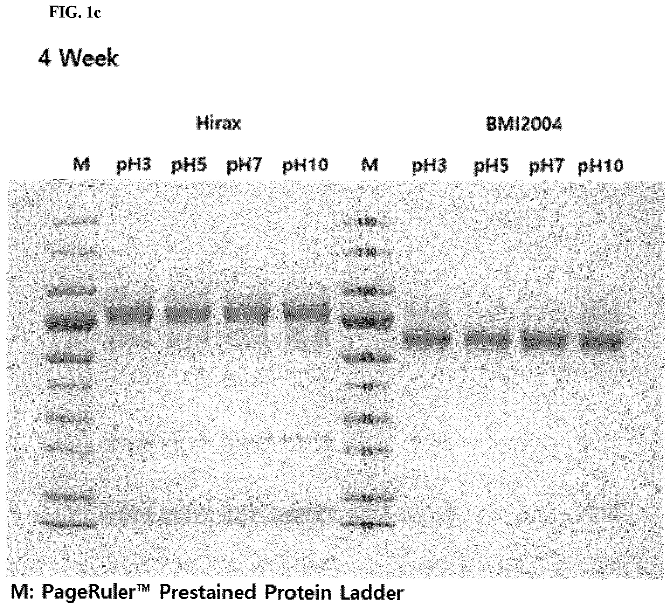

The test results are depicted in FIG. 1a to FIG. 1c. As shown in FIG. 1a to FIG. 1c, it could be understood from whether or not other band is generated according to time flow in the state of being replaced with water on SDS-PAGE results that BMI2004 was more stable against pH with time than Hirax.

**(2) Comparison of enzyme activity or content according to storage temperature**

Comparison was made of pH stability between BMI2004 prepared in Example 1 and Hirax. The BMI2004 and Hirax used in the temperature stability test were 95% or higher in purity and each had a concentration of 1,500 IU/mL (=100%). Solutions were prepared using water according to the conditions in Table 7 and stored at a frozen temperature (-20°C), a refrigerated temperature (5°C), and a high temperature (40°C) for 4 weeks during which a titer test was conducted every two weeks.

TABLE 7

| Storage Temp. | Specimen | Storage pH |
|---|---|---|
| ≤-20 °C | | pH 5.0 |
| 2-8 °C | Hirax / BMI2004 (1,500 IU/mL) | pH 5.0 |
| ≥40 °C | | pH 5.0 |

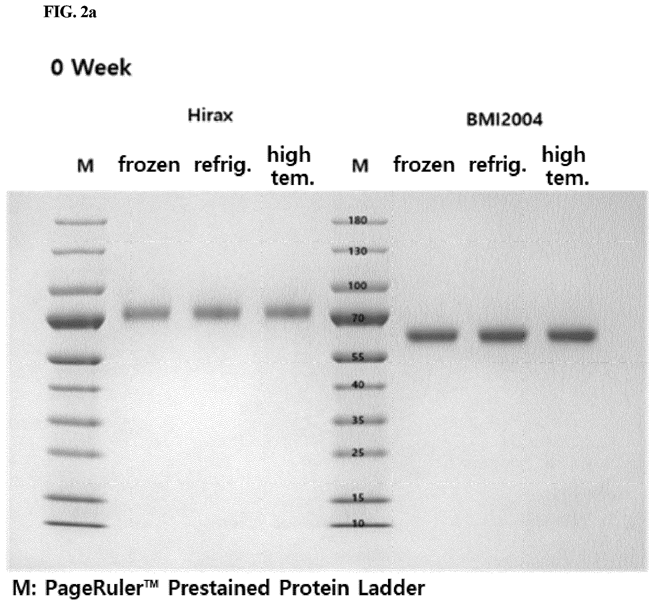

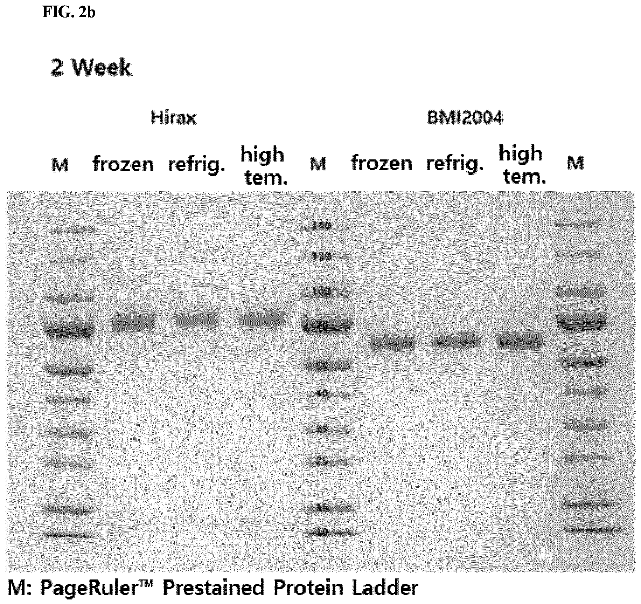

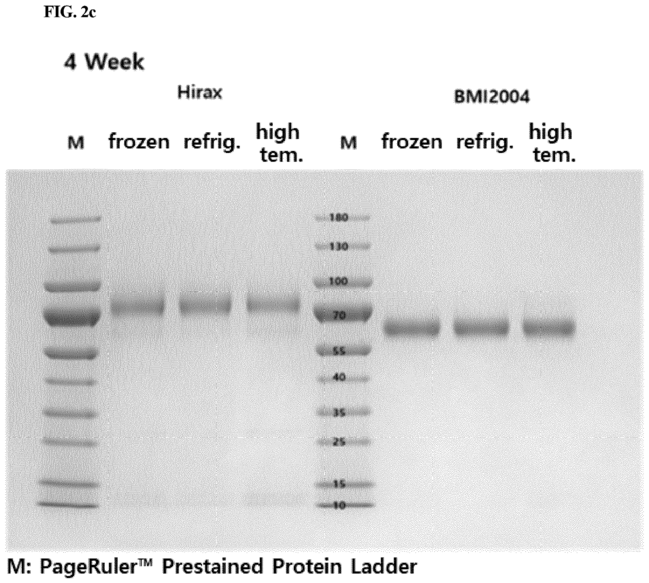

Hirax and BMI2004 were measured for stability against temperature for 4 weeks and the measurements are summarized in Table 8 and depicted in FIG. 2. The highest contents (%) were detected at frozen temperatures of -18°C or less. After week 2, the content (%) of BMI2004 was maintained significantly high relative to Hirax in all temperature conditions.

TABLE 8

| Enzyme activity ratio (%) | Frozen | | | Refrigerated | | | High temperature | | |
|---|---|---|---|---|---|---|---|---|---|
| Specimen | Week 0 | Week 2 | Week 4 | Week 0 | Week 2 | Week 4 | Week 0 | Week 2 | Week 4 |
| BMI2004 | 102.68 | 100.88 | 99.22 | 102.77 | 101.91 | 100.23 | 99.47 | 89.90 | 79.63 |

(continued)

| Enzyme activity ratio (%) | Frozen | | | Refrigerated | | | High temperature | | |
|---|---|---|---|---|---|---|---|---|---|
| Specimen | Week 0 | Week 2 | Week 4 | Week 0 | Week 2 | Week 4 | Week 0 | Week 2 | Week 4 |
| Hirax | 102.76 | 80.24 | 59.75 | 101.42 | 91.51 | 82.83 | 102.19 | 66.40 | 40.15 |

In an additional stability assay according to temperature, Hirax and BMI2004 were measured for content changes at various temperature in the same manner as in Example 3. In this regard, SDS-PAGE was conducted in the conditions of Table 7. Results of SDS-PAGE are depicted in FIG. 2. As shown in FIG. 2, it was understood from the formation of other bands positioned other than the initial position of the main band at frozen, refrigerated, and high temperatures with time in an aqueous condition, that BMI2004 was more stable than Hirax.

**EXAMPLE 4. Optimal Enzyme Activity According to Temperature**

A plate titer assay in this Example was designed on the basis of the cylinder plate assay among microbial assays for antibiotics. Specifically, peni-cylinders having a predetermined amount of hyaluronidase distributed therein were placed on solid agarose plates containing hyaluronic acid to allow the hyaluronidase to diffuse across the agarose plates. Through this diffusion, the hyaluronic acid was degraded by hyaluronidase, and the hyaluronic acid remaining undegraded were precipitated by cetylpyridinium chloride to form clear halos. In this Example, the enzymatic activity of hyaluronidase could be thus determined by the size of the halo generated through such a simple method using plates. In each condition, the plate titer assay was conducted as follows:

1) Preparation of hyaluronic acid solution: 0.2 g of hyaluronic acid was completely dissolved in 100 mL of water and the pH was adjusted with hydrochloric acid and sodium hydroxide to a pH of $7.0\pm0.1$.
2) Preparation of 1.5% agarose: 1.5 g of agarose (SIGMA, Cat No. A9539) was added to 100 mL of water and dissolved with microwaves.
3) Preparation of 10% cetylpyridinium chloride: 10 g of cetylpyridinium chloride (SIGMA, Cat No. C0732) was dissolved in 100 mL of water.
4) Test solution: Hirax and BMI2004 reference standards were adjusted with water according to the conditions.
5) Manual: Test solutions were subjected to the following assays.
5-1) The hyaluronic acid solution was warmed at 37°C for about 20 minutes before mixing with 1.5% agarose.
5-2) After 100 mL of 1.5% agarose was cooled to about 60°C, 100 mL of the hyaluronic acid solution was added and mixed by stirring.
5-3) The mixture was poured to a thickness of about 3 mm in a petri dish (SPL, Cat No. 10050).
5-4) After the agarose gel was fully solidified, the peni cylinder (KisanBio, Cat No. KS-P0161) was placed at an angle of 90° on the concentric circle of the petri dish. The test solution was taken in an amount of 20 μL and loaded into the peni cylinder, followed by incubation for 18-20 hours in an incubator maintained at the temperature conditions listed in each table.
5-5) After completion of the reaction, the peni cylinder was removed and 3 mL of 10% cetylpyridinium chloride was added. After 20 minutes, appearance of clear halos was monitored.
5-6) The halo was measured precisely to an extent of 0.5 mm or less for diameter (mm).

**(1) Confirmation of Activity of hyaluronic acid solution**

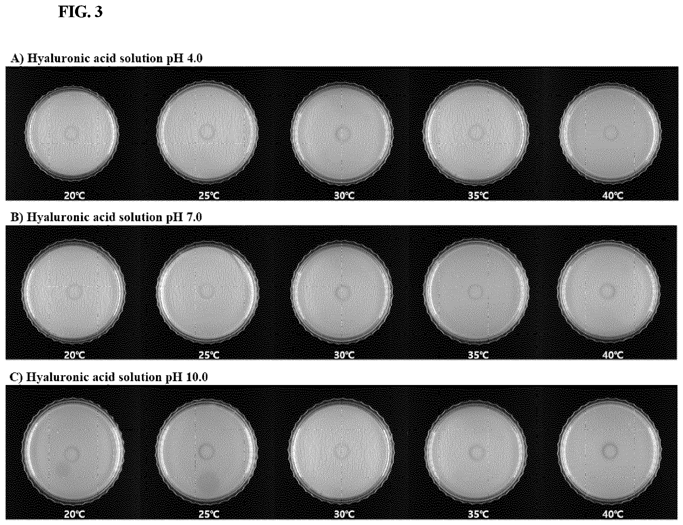

In order to examine whether hyaluronic acid solutions exhibited their own activity, plate titer tests were carried out at various pH values and temperature conditions before an assay for optimal enzymatic activity. The tests were carried out at 20°C, 25°C, 30°C, 35°C, and 40°C under the condition of pH 4.0, pH 7.0, or pH 10.0. The test results are depicted in FIG. 3. As shown in FIG. 3, the hyaluronic acid solutions were observed to have no their own activity.

**(2) Comparison of enzymatic activity in temperature condition of 20-40°C**

The BMI2004 and Hirax used in this assay for enzymatic activity were 95% or higher in purity and were each adjusted with water to have a concentration of 1,500 IU/mL (=100%). Solutions were prepared according to the conditions in Table 9 and subjected to plate titer assays at 20°C, 25°C, 30°C, 35°C, 40°C, and 60°C.

TABLE 9

|  | Storage pH | Storage Temp. | Specimen |
|---|---|---|---|
| Condition 1 | pH 4.0 | 20-40 °C | |
| Condition 2 | pH 7.0 | 20-40 °C | Hirax / BMI2004 (1,500 IU/mL) |
| Condition 3 | pH 10.0 | 20-40 °C | |

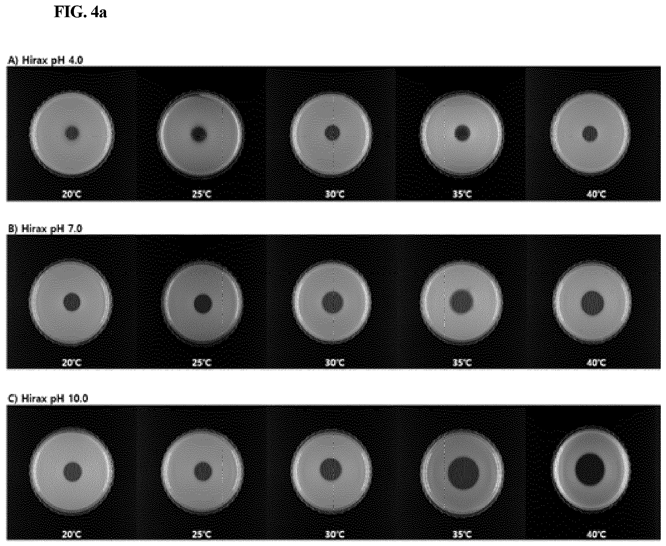

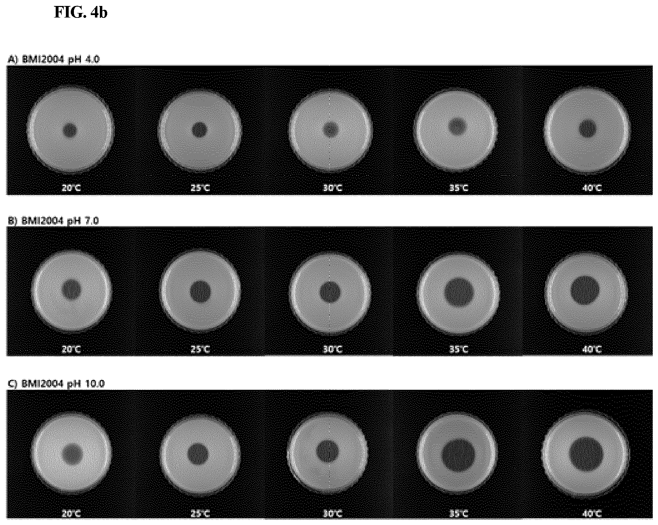

Assay results are depicted in FIG. 4a and FIG. 4b. Enzymatic activity of Hirax and BMI2004 was examined by a plate titer assay at 20-60°C. As a result, the halos appeared large at 35°C and 40°C. To quantitate enzymatic activity, diameters of the halos in FIG. 4a and FIG. 4b were measured and the measurements are shown in Table 10, below. In Table 10, the diameters (mm) had an error deviation of $\pm 0.5$ mm.

TABLE 10

| Temp. | Specimen | pH 4.0 | pH 7.0 | pH 10.0 |
|---|---|---|---|---|
| 20 | Hirax | 8 mm | 10 mm | 11 mm |
|  | BMI2004 | 8 mm | 12 mm | 13 mm |
| 25 | Hirax | 9 mm | 10 mm | 11 mm |
|  | BMI2004 | 9 mm | 13 mm | 13 mm |
| 30 | Hirax | 9 mm | 13 mm | 14 mm |
|  | BMI2004 | 10 mm | 13 mm | 15 mm |
| 35 | Hirax | 10 mm | 15 mm | 18 mm |
|  | BMI2004 | 11 mm | 18 mm | 21 mm |
| 40 | Hirax | 10 mm | 15 mm | 18 mm |
|  | BMI2004 | 11 mm | 18 mm | 21 mm |

As shown in Table 10, comparison of enzymatic activity at 20 to 40°C indicated that the activity of BMI2004 was higher than that of Hirax.

**(3) Comparison of enzymatic activity in temperature condition of 35-40°C**

On the basis of the results of activity comparison between Hirax and BMI2004 measured in (2) of Example 4, additional tests were conducted in subdivided temperature conditions. In this regard, solutions were prepared according to the conditions listed in Table 11 below and subjected to plate titer assays at 35°C, 37°C, and 40°C.

TABLE 11

|  | Storage pH | Storage Temp. | Specimen |
|---|---|---|---|
| Condition 1 | pH 4.0 | 35°C | |
|  |  | 37°C | |
|  |  | 40°C | |
| Condition 2 | pH 7.0 | 35°C | Hirax / BMI2004 (1,500 IU/mL) |
|  |  | 37°C | |
|  |  | 40°C | |
| Condition 3 | pH 10.0 | 35°C | |
|  |  | 37°C | |
|  |  | 40°C | |

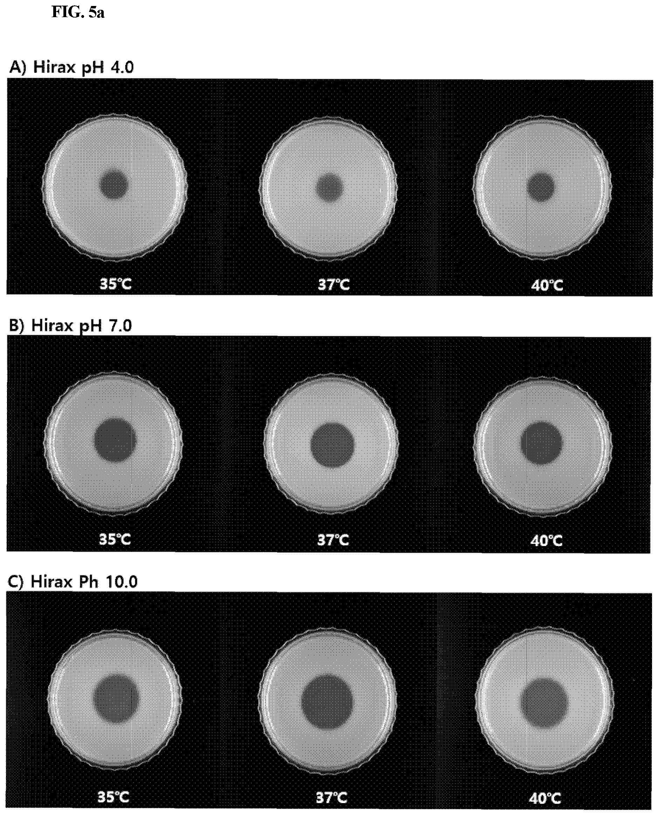

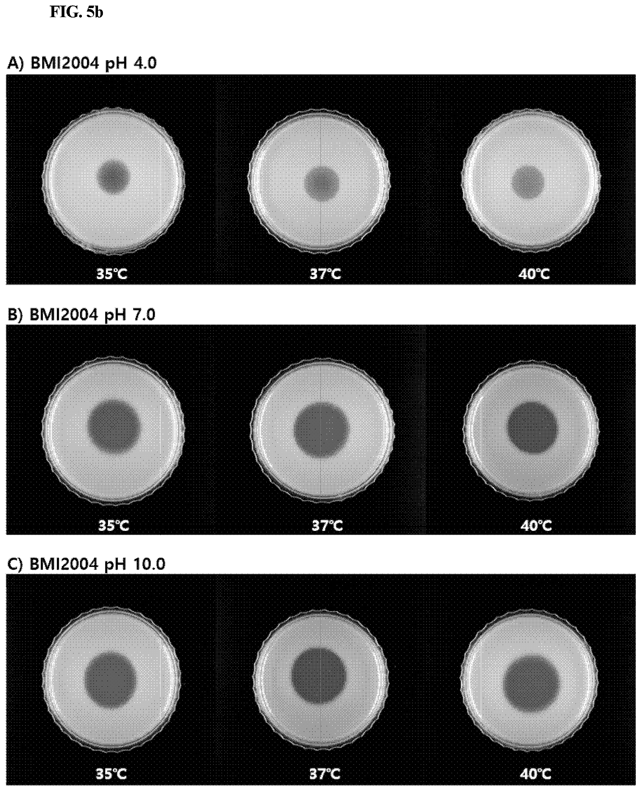

The test results are shown in FIG. 5a and FIG. 5b. Enzymatic activity of Hirax and BMI2004 was examined by a plate titer assay at 35°C, 37°C, and 40°C. As a result, the halos appeared largest at 37°C. Diameters of the halos were shown in Table 12, below. The diameters (mm) had an error deviation of ±0.5 mm.

TABLE 12

| Specimen | 35°C | | 37°C | | 40°C | |
|---|---|---|---|---|---|---|
| | Hirax | BMI2004 | Hirax | BMI2004 | Hirax | BMI2004 |
| pH 4.0 | 10 mm | 12 mm | 10 mm | 13 mm | 10 mm | 12 mm |
| pH 7.0 | 16 mm | 19 mm | 17 mm | 20 mm | 16 mm | 19 mm |
| pH 10.0 | 18 mm | 21 mm | 19 mm | 22 mm | 18 mm | 21 mm |

As shown in Table 12, comparison of enzymatic activity at 35°C, 37°C, and 40°C indicated that the activity of BMI2004 was higher than that ofHirax.

**EXAMPLE 5. Optimal Enzymatic Activity According to pH**

The Hirax and BMI2004 used in the assay for optimal enzymatic activity were 95% or higher in purity and were each adjusted with water to have a concentration of 1,500 IU/mL (=100%). Solutions were prepared according to the conditions in Table 13. The enzymatic activity was measured according to the assay of Example 4. On the basis of the activity result according to temperature measured in (3) of Example 4, a plate titer assay according to pH was conducted in the condition of 37°C at which the largest halo appeared.

TABLE 13

| | Storage pH | Storage Temp | Specimen |
|---|---|---|---|
| Condition 3 | pH 5.0 | | |
| Condition 4 | pH 6.0 | | |
| Condition 5 | pH 7.0 | 37 °C | Hirax / BMI2004 (1,500 IU/mL) |
| Condition 6 | pH 8.0 | | |
| Condition 7 | pH 9.0 | | |
| Condition 8 | pH 10.0 | | |

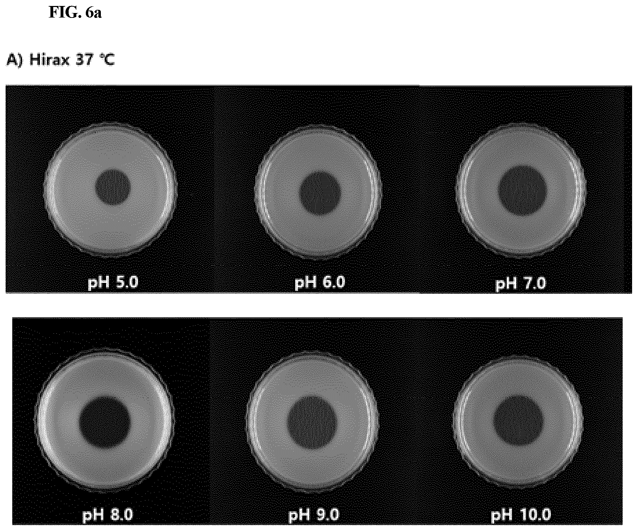

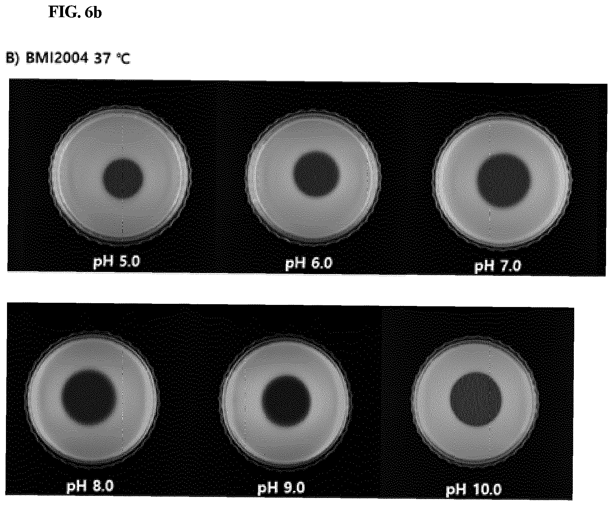

Assay results are depicted in FIG. 6a and FIG. 6b. As a result of the plate titer assay for enzymatic activity of Hirax and BMI2004 at 37°C under the condition of pH 5.0-10.0, the halos appeared particularly large at a pH of 7.0-10.0. Comparison of halo diameters are shown in Table 14 below. The diameter (mm) had an error deviation of ±0.5 mm.

TABLE 14

| Specimen | 37 °C | |
|---|---|---|
| | Hirax | BMI2004 |
| pH 5.0 | 14 mm | 16 mm |
| pH 6.0 | 17 mm | 18 mm |
| pH 7.0 | 19 mm | 20 mm |
| pH 8.0 | 19 mm | 20 mm |
| pH 9.0 | 19 mm | 20 mm |
| pH 10.0 | 20 mm | 21 mm |

As can be seen in Table 14, comparison of enzymatic activity at 37°C under the condition of pH 3.0-10.0 indicated that the activity of BMI2004 was superior to that of Hirax at a pH of 5.0 or higher, and particularly superior at a pH of 7.0 or higher.

**EXAMPLE 6. Assay for Titer of Hyaluronidase**

The hyaluronidase according to an embodiment of the present disclosure and the conventional enzyme Hirax were measured for titer in the same manner as in (1) of Example 2, and the measurements are shown in Table 15.

TABLE 15

| Sample no. | BMI2004 (IU/mg) | Hirax (IU/mg) |
|---|---|---|
| 1 | 124,497 | 81,396 |
| 2 | 133,870 | 81,528 |
| 3 | 134,678 | 80,653 |
| 4 | 122,433 | 80,538 |
| Average | 128,870 | 81,029 |

As shown in Table 15, BMI2004 exhibited an activity of about 122,433 to 134,678 IU per mg of protein while Hirax exhibited an activity of about 80,538 to 81,396 IU per mg of protein. Therefore, the hyaluronidase according to an embodiment of the present disclosure could achieve equivalent activity even when used in an amount of about 63 % of the amount of conventional Hirax.

**EXAMPLE 7. Promotive Effect of Hyaluronidase on Drug Absorption and Diffusion (1)**

In order to confirm the promotive effect on drug absorption and diffusion of the hyaluronidase according to an embodiment of the present disclosure, an assay for drug absorption and diffusion promotion was carried out using Trypan Blue.

Specifically, Hirax and BMI2004 were each mixed with 0.2% Trypan blue solution and injected subcutaneously once into Balb/c nude mice which were monitored for the diffusion of trypan blue. On the basis of the titers listed in Table 15, all the drugs were formulated to have a concentration or 10 IU/mL or 100 IU/mL under the conditions of Table 16 and administered at a dose of 0.05 mL. At each time, the diffused areas ($\pi mm^2$) were measured.

TABLE 16

| Specimen (IU/mL) | Peptide dose (ug/mL) | 2.5 min | 5 min | 10 min | 15 min | 20 min |
|---|---|---|---|---|---|---|
| Saline | 0 | 77.38 ±15.86 | 84.61±13.92 | 98.11±7.83 | 117.67±10.35 | 121.84±11.48 |
| Hirax (10 IU/mL) | about 0.123 | 74.45 ±22.40 | 112.84±15.01 * | 146.69±29.36** | 165.98±18.49* | 189.90±41.85** |
| Hirax (100 IU/mL) | about 1.234 | 84.25 ±15.81 | 133.90 ±14.52*** | 149.27±27.41** | 184.80±37.10** | 215.12±20.60*** |
| BMI2004 (10 IU/mL) | about 0.078 | 82.70 ±22.95 | 109.65±12.70* | 153.17 ±15.30*** | 176.85±14.08** | 187.63±36.40** |
| BMI2004 (100 IU/mL) | about 0.776 | 87.02 ±17.51 | 126.70 ±16.00*** | 140.23 ±16.95*** | 180.98 ±29.93*** | 218.14±26.85*** |
| 1) Mean±SD | | | | | | |
| 2) *p < 0.05, **p < 0.01, ***p < 0.001 compared to saline treated group by SPSS (one-way ANOVA, LSD test) | | | | | | |

As shown in Table 16, the diffused areas for 2.5 to 20 minutes were measured. From 5 minutes after administration, Hirax and BMI2004 significantly increased the diffused areas compared to the negative control saline.

**EXAMPLE 8. Promotive Effect of Hyaluronidase on Drug Absorption and Diffusion (2)**

In order to confirm the promotive effect on drug absorption and diffusion of the hyaluronidase according to an embodiment of the present disclosure, an assay for drug absorption and diffusion promotion was carried out using an akinesia model.

Specifically, Cynomolgus Monkeys were used as Akinesia Models for examining ability to increase the penetration of drugs. Test materials were BMI2004 and Hirax listed in Table 15 while saline was used as a negative control. Lidocaine and bupivacaine were used as anesthetics to be mixed with the specimens. The specimens were each injected at a dose of 2 mL to 6 monkeys through the right and the left peribulbar route.

After administration of the anesthetics along with the specimens, the time until the movement of the pupil disappeared (time to akinesia) and the time from the anesthesia of the pupil to the observation of pupil movement (duration of akinesia) were measured and the measurements are shown in Table 17.

TABLE 17

| Specimen | Peptide dose (ug/mL) | Model (Monkey) | Eye | Time to Akinesia (min) | Duration of Akinesia (min) |
|---|---|---|---|---|---|
| Saline | 0 | 02 | Left | No Akinesia | No Akinesia |
| | | 03 | Right | No Akinesia | No Akinesia |
| | | 05 | Left | No Akinesia | No Akinesia |
| | | 06 | Right | No Akinesia | No Akinesia |
| Hirax (500 IU/ 0.2 mL) | ca. 6.17 $\mu$g/ 0.2mL | 01 | Left | 4 | 27 |
| | | 02 | Right | 4 | 27 |
| | | 04 | Left | 2 | 17 |
| | | 05 | Right | 2 | 32 |
| | | Mean | | 3.0 | 25.8 |
| | | SD | | 1.2 | 6.3 |
| BMI2004 (500 IU/ 0.2 mL | ca. 3.88 $\mu$g/ 0.2mL | 01 | Right | 1 | 32 |
| | | 03 | Left | 2 | 24 |
| | | 04 | Right | 2 | 17 |
| | | 06 | Left | 1 | 41 |
| | | Mean | | 1.5 | 28.5 |
| | | SD | | 0.6 | 10.3 |
| 1) Drug; Lidocaine 2% (0.9 mL), Bupivacaine 0.5% (0.9 mL) and the dosing article (0.2 mL) | | | | | |

As seen in Table 17, the negative control saline exhibited no akinesia effects, while when administered together with the anesthetics, the peptide according to an embodiment of the present disclosure enhanced the penetration of the anesthetics and thus brought about an akinesia effect equivalent to that of the conventional peptide Hirax, demonstrating its ability to induce drug absorption and diffusion. In addition, the peptide according to an embodiment of the present disclosure exhibited equivalent activity at a protein dose of about 63% of that of conventional Hirax.

**EXAMPLE 9. Assay for Ability of Hyaluronidase to Promote Reabsorption of Excessive Body Fluid**

In order to confirm the promotive effect on reabsorption of excessive body fluid, an edema model was employed. In this Example, the edema model was constructed by artificially causing lymphedema therein. Test materials were BMI2004 and Hirax listed in Table 15 while saline was used as a negative control. Specimens and doses are given in Table 18 below.

TABLE 18

| Specimen | Lymphedema | Dose (/site) | Route | No. of animal |
|---|---|---|---|---|
| Saline | + | - | SC | 6 |
| Hirax | + | 100 IU (ca. 1.234ug) | SC | 6 |
| BMI2004 | + | 100 IU (ca. 0.776ug) | SC | 6 |

Lymphedema induction was conducted in the tail due to accuracy and ease. An annular cut of the skin 2 mm wide was made 1 cm from the base of the tail of the mouse. In this regard, about 4 mm$^2$ of the ventral side was left without being cut out. In order to examine the effect of reducing edema, as listed in Table 19 below, the diameter (mm) of the tail was measured at each time point from pre-administration (0 hour) to day 15 of induction after the first administration. The dose was made secondarily 24 hours later and thirdly 48 hours later. In this test, the diameter (mm) of the tail was measured using a caliper at the 10 mm point of the defect site.

TABLE 19

| Specimen | Saline | Hirax | BMI2004 |
|---|---|---|---|
| 0 hr | 4.55±0.43 | 4.53±0.49 | 4.53±0.38 |
| 1 hr | 4.57±0.43 | 4.39±0.49 | 4.57±0.65 |
| 2 hrs | 4.48±0.36 | 4.45±0.40 | 4.50±0.37 |
| 4 hrs | 4.52±0.38 | 4.41±0.38 | 4.48±0.51 |
| 24 hrs | 4.56±0.34 | 4.57±0.39 | 4.55±0.45 |
| 25 hrs | 4.68±0.32 | 4.48±0.35 | 4.53±0.44 |
| 26 hrs | 4.56±0.32 | 4.43±0.38 | 4.45±0.47 |
| 28 hrs | 4.58±0.37 | 4.42±0.44 | 4.46±0.48 |
| 48 hrs | 4.65±0.39 | 4.49±0.41 | 4.47±0.38 |
| 49 hrs | 4.56±0.27 | 4.22±0.37 | 4.26±0.37 |
| 50 hrs | 4.58±0.25 | 4.13±0.38 | 4.09±0.36 |
| 52 hrs | 4.59±0.25 | 4.16±0.47 | 4.18±0.36 |

As shown in Table 19, the administration of BMI2004 reduced lymphedema, demonstrating the promotive effect of the protein on reabsorption of excessive body fluids. In addition, the peptide according to an embodiment of the present invention exhibited equivalent activity at a protein dose of about 63% compared to conventional Hirax.

**EXAMPLE 10. Assay for In-vivo Stability of Hyaluronidase**

In order to confirm in-vivo stability, the hyaluronidase according to an embodiment of the present disclosure was intravenously injected and monitored for pharmacokinetics. In this Example, SD-rats were used as test animals. To the animals, Hirax and BMI2004 were each administered at a total dose of about 180,000 IU for 30 minutes by intravenous infusion. Blood samples were taken before administration, 15 minutes after initiation of the infusion (mid-infusion), 30 minutes after initiation of the infusion (end of infusion), and 31, 33, 36, 40, 45, 60, 75, 90 minutes, and 2.5 hours, 4.5 hours, 24.5 hours, and 48.5 hours after initiation of the infusion.

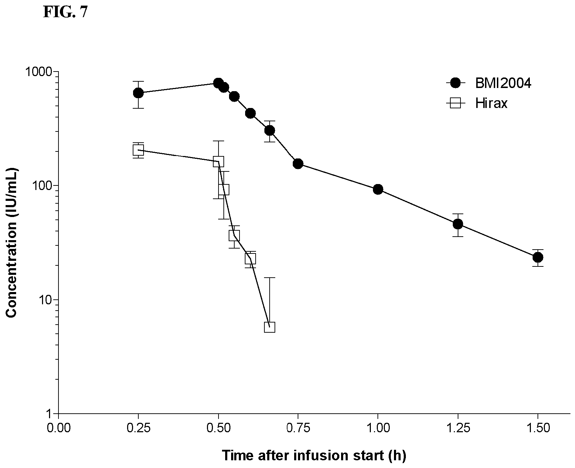

Pharmacokinetic variables of the polypeptide according to an embodiment of the present disclosure and Hirax are given in Table 20. The blood levels of the polypeptide over time are depicted in FIG. 7. As seen in FIG. 7 and Table 20, Hirax was degraded too rapidly to measure in-vivo half-life. In contrast, the peptide according to an embodiment of the present disclosure was observed to have a half-life of about 0.272 hours (about 16.3 minutes). With a longer half-life than Hirax, the polypeptide according to an embodiment of the present disclosure has the potential to show a greater effect than Hirax by staying in the body for a long time. In addition, they were different in terms of in-vivo blood level. The blood level of Hirax peaked within 0.25 hours (15 minutes), but the blood level of BMI2004 peaked at 0.5 hours (30 minutes) after administration. The polypeptide exhibited a potential to bring about a greater effect than Hirax as it more slowly increased in blood level and remained in vivo for a longer period of time than Hirax.

TABLE 20

| Test material | Dose (IU/kg) | $t_{max}$(h) | $t_{1/2}$(h) | CL (mL/h/kg) |
|---|---|---|---|---|
| Hirax | 180000 | 0.250 | NC | NC |
| BMI2004 | 180000 | 0.500 | 0.272 | 418 |

**Claims**

1. A polypeptide having hyaluronidase activity, which includes deletion of at least one consecutive amino acid of 1 to 203 amino acids from a C-terminus of a polypeptide consisting of the amino acid sequence of SEQ ID NO: 1.

2. The polypeptide of claim 1, including deletion of at least one consecutive amino acid of 34 to 170 amino acids from the C-terminus of the amino acid sequence of SEQ ID NO: 1.

3. The polypeptide of claim 1, including deletion of at least one consecutive amino acid of 34 to 68 amino acids from the C-terminus of the amino acid sequence of SEQ ID NO: 1.

4. The polypeptide of claim 1, including deletion of an amino acid sequence of 34, 68, 102, 136, or 170 amino acids from the C-terminus of the amino acid sequence of SEQ ID NO: 1.

5. The polypeptide of claim 1, including further deletion of a first amino acid from the N-terminus of the amino acid sequence of SEQ ID NO: 1.

6. The polypeptide of claim 1, wherein the polypeptide consists of an amino acid sequence of SEQ ID NO: 2, SEQ ID NO: 3, SEQ ID NO: 4, SEQ ID NO: 5, or SEQ ID NO: 6.

7. The polypeptide of claim 1, wherein the polypeptide is glycosylated.

8. The polypeptide of claim 1, wherein the polypeptide is stable at pH 3 to 10.

9. The polypeptide of claim 1, wherein the polypeptide is stable at a temperature of -20 to 45°C.

10. The polypeptide of claim 1, wherein the polypeptide has an enzymatic activity of 57% or higher of an initial activity after storage at pH condition in the range between higher than pH 3 and pH 10 or less for 4 weeks.

11. The polypeptide of claim 1, wherein the polypeptide has an enzymatic activity of 32% or higher of an initial activity after storage at pH condition in the range between pH 3 or higher and less than pH 5 for 4 weeks.

12. The polypeptide of claim 1, wherein the polypeptide has an enzymatic activity of 63% or higher of an initial activity thereof after storage at a temperature less than 0°C for 4 weeks.

13. The polypeptide of claim 1, wherein the polypeptide has an enzymatic activity of 83% or higher of an initial activity thereof after storage at a temperature of 0 to 40°C for 4 weeks.

14. The polypeptide of claim 1, wherein the polypeptide has an enzymatic activity of 52% or higher of an initial activity thereof after storage at a temperature of 40°C or more for 4 weeks.

15. The polypeptide of claim 1, wherein the polypeptide has an activity in the range between higher than one-fold and three-folds or less of that of the polypeptide consisting of an amino acid sequence of SEQ ID NO: 1.

16. The polypeptide of claim 1, wherein the polypeptide has an activity of 120,000 to 150,000 IU/mg.

17. A nucleic acid molecule, encoding the polypeptide of any one of claims 1 to 16.

18. A vector, comprising the nucleic acid molecule of claim 17.

19. A cell, comprising the vector of claim 18.

20. The cell of claim 19, wherein the cell is selected from the group consisting of bacteria, yeasts, fungi, insect cells, animal cells, mammalian cells, algal cells, and plant cells.

21. The cell of claim 20, wherein the mammalian cells are selected from the group consisting of CHO, NS0, HEK293, BHK, Per.C6, MDCK, Vero, MRC, HeLa, IMR, and Sp2/0.

22. A composition for topical administration, comprising the polypeptide of any one of claims 1 to 16.

23. The composition of claim 22, wherein the composition is adapted for subcutaneous administration or intramuscular injection.

24. A drug delivery carrier, comprising the polypeptide of any one of claims 1 to 16.

25. A composition for prevention or treatment of edema, comprising the polypeptide of any one of claims 1 to 16.

**FIG. 1a**

## 0 Week

M: PageRuler™ Prestained Protein Ladder

**FIG. 1b**

## 2 Week

M: PageRuler™ Prestained Protein Ladder

**FIG. 1c**

## 4 Week

M: PageRuler™ Prestained Protein Ladder

**FIG. 2a**

0 Week

M: PageRuler™ Prestained Protein Ladder

**FIG. 2b**

**2 Week**

M: PageRuler™ Prestained Protein Ladder

FIG. 2c

M: PageRuler™ Prestained Protein Ladder

**FIG. 3**

A) Hyaluronic acid solution pH 4.0

B) Hyaluronic acid solution pH 7.0

C) Hyaluronic acid solution pH 10.0

**FIG. 4a**

A) Hirax pH 4.0

B) Hirax pH 7.0

C) Hirax pH 10.0

**FIG. 4b**

A) BMI2004 pH 4.0

B) BMI2004 pH 7.0

C) BMI2004 pH 10.0

**FIG. 5a**

A) Hirax pH 4.0

B) Hirax pH 7.0

C) Hirax Ph 10.0

**FIG. 5b**

A) BMI2004 pH 4.0

B) BMI2004 pH 7.0

C) BMI2004 pH 10.0

**FIG. 6a**

A) Hirax 37 ℃

**FIG. 6b**

B) BMI2004 37 °C

**FIG. 7**

| | INTERNATIONAL SEARCH REPORT | International application No. |
|---|---|---|
| | | **PCT/KR2022/011586** |

**A.    CLASSIFICATION OF SUBJECT MATTER**

**C12N 9/26**(2006.01)i; **A61K 38/47**(2006.01)i; **A61P 7/10**(2006.01)i

According to International Patent Classification (IPC) or to both national classification and IPC

**B.    FIELDS SEARCHED**

Minimum documentation searched (classification system followed by classification symbols)

C12N 9/26(2006.01); A61K 31/337(2006.01); A61K 38/16(2006.01); A61K 38/47(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 히알루로니다제(hyaluronidase), 안전성(stability), PH20

**C.    DOCUMENTS CONSIDERED TO BE RELEVANT**

| Category* | Citation of document, with indication, where appropriate, of the relevant passages | Relevant to claim No. |
|---|---|---|
| A | KR 10-2021-0023798 A (ALTEOGEN, INC.) 04 March 2021 (2021-03-04)<br>       See abstract; paragraph [0001]; and table 11. | 1-25 |
| A | NCBI Reference Sequence: XP_027824574.1 (03 July 2021).<br>       See entire document. | 1-25 |
| A | NCBI Reference Sequence: XP_043319552.1 (06 September 2021).<br>       See entire document. | 1-25 |
| A | WO 2013-102144 A2 (HALOZYME, INC.) 04 July 2013 (2013-07-04)<br>       See entire document. | 1-25 |
| A | US 2010-0143457 A1 (WEI, G. et al.) 10 June 2010 (2010-06-10)<br>       See entire document. | 1-25 |

☐ Further documents are listed in the continuation of Box C.       ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 March 2023** | **06 March 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2022/011586**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

          ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

## INTERNATIONAL SEARCH REPORT
### Information on patent family members

| International application No. |
| --- |
| **PCT/KR2022/011586** |

| Patent document cited in search report | | | Publication date (day/month/year) | Patent family member(s) | | | Publication date (day/month/year) |
| --- | --- | --- | --- | --- | --- | --- | --- |
| KR | 10-2021-0023798 | A | 04 March 2021 | AU | 2019-311658 | A1 | 30 January 2020 |
| | | | | AU | 311658 | B2 | 20 October 2022 |
| | | | | BR | 112020019041 | A2 | 09 February 2021 |
| | | | | CA | 3093885 | A1 | 30 January 2020 |
| | | | | CN | 111971387 | A | 20 November 2020 |
| | | | | EP | 3636752 | A1 | 15 April 2020 |
| | | | | JP | 2021-507676 | A | 25 February 2021 |
| | | | | JP | 7204729 | B2 | 16 January 2023 |
| | | | | KR | 10-2020-0017538 | A | 18 February 2020 |
| | | | | KR | 10-2151388 | B1 | 04 September 2020 |
| | | | | RU | 2766680 | C1 | 15 March 2022 |
| | | | | US | 2021-0155913 | A1 | 27 May 2021 |
| | | | | WO | 2020-022791 | A1 | 30 January 2020 |
| WO | 2013-102144 | A2 | 04 July 2013 | AU | 2012-362141 | A1 | 10 July 2014 |
| | | | | AU | 362141 | B2 | 21 September 2017 |
| | | | | BR | 112014016195 | A2 | 27 October 2020 |
| | | | | CA | 2861919 | A1 | 04 July 2013 |
| | | | | CA | 2861919 | C | 02 April 2019 |
| | | | | CN | 104244968 | A | 24 December 2014 |
| | | | | CN | 104244968 | B | 25 July 2017 |
| | | | | DK | 2797622 | T3 | 16 January 2017 |
| | | | | DK | 3130347 | T3 | 14 October 2019 |
| | | | | EA | 030252 | B1 | 31 July 2018 |
| | | | | EA | 030252 | B9 | 08 September 2021 |
| | | | | EA | 201400772 | A1 | 30 July 2015 |
| | | | | EP | 2797622 | A2 | 05 November 2014 |
| | | | | EP | 2797622 | B1 | 12 October 2016 |
| | | | | EP | 3130347 | A1 | 15 February 2017 |
| | | | | EP | 3130347 | B1 | 18 September 2019 |
| | | | | ES | 2609582 | T3 | 21 April 2017 |
| | | | | ES | 2749620 | T3 | 23 March 2020 |
| | | | | HK | 1202814 | A1 | 09 October 2015 |
| | | | | HR | P20192249 | T1 | 06 March 2020 |
| | | | | HU | E047849 | T2 | 28 May 2020 |
| | | | | IL | 233192 | A | 30 June 2020 |
| | | | | IL | 233192 | B | 30 June 2020 |
| | | | | IL | 274798 | A | 30 July 2020 |
| | | | | IL | 274798 | B | 25 March 2021 |
| | | | | IL | 280949 | A | 29 April 2021 |
| | | | | IL | 280949 | B | 01 December 2022 |
| | | | | JP | 2015-504666 | A | 16 February 2015 |
| | | | | JP | 2017-112999 | A | 29 June 2017 |
| | | | | JP | 6067746 | B2 | 25 January 2017 |
| | | | | JP | 6422933 | B2 | 14 November 2018 |
| | | | | LT | 3130347 | T | 25 October 2019 |
| | | | | MX | 2014007966 | A | 17 October 2014 |
| | | | | MX | 361727 | B | 14 December 2018 |
| | | | | NZ | 626126 | A | 24 June 2016 |
| | | | | NZ | 720075 | A | 27 March 2020 |
| | | | | PL | 3130347 | T3 | 30 April 2020 |

Form PCT/ISA/210 (patent family annex) (July 2022)

**INTERNATIONAL SEARCH REPORT**
Information on patent family members

| International application No. |
| --- |
| **PCT/KR2022/011586** |

| Patent document cited in search report | | | Publication date (day/month/year) | Patent family member(s) | | | Publication date (day/month/year) |
| --- | --- | --- | --- | --- | --- | --- | --- |
| | | | | PT | 3130347 | T | 10 December 2019 |
| | | | | RS | 59703 | B1 | 31 January 2020 |
| | | | | SG | 10201604470 | A | 28 July 2016 |
| | | | | SG | 11201403714 | A | 30 July 2014 |
| | | | | SI | 3130347 | T1 | 28 February 2020 |
| | | | | US | 10865400 | B2 | 15 December 2020 |
| | | | | US | 11041149 | B2 | 22 June 2021 |
| | | | | US | 11066656 | B2 | 20 July 2021 |
| | | | | US | 2013-0302275 | A1 | 14 November 2013 |
| | | | | US | 2016-0362670 | A1 | 15 December 2016 |
| | | | | US | 2020-0255814 | A1 | 13 August 2020 |
| | | | | US | 2020-0318091 | A1 | 08 October 2020 |
| | | | | US | 2021-0277376 | A1 | 09 September 2021 |
| | | | | US | 2021-0284985 | A1 | 16 September 2021 |
| | | | | US | 9447401 | B2 | 20 September 2016 |
| | | | | WO | 2013-102144 | A3 | 06 February 2014 |
| US | 2010-0143457 | A1 | 10 June 2010 | AU | 2009-333918 | A1 | 07 July 2011 |
| | | | | AU | 333918 | B2 | 05 June 2014 |
| | | | | BR | PI0922538 | A2 | 13 October 2020 |
| | | | | CA | 2746181 | A1 | 08 July 2010 |
| | | | | CA | 2746181 | C | 15 March 2016 |
| | | | | CA | 2915783 | A1 | 08 July 2010 |
| | | | | CA | 2915783 | C | 21 July 2020 |
| | | | | CN | 102307993 | A | 04 January 2012 |
| | | | | CN | 102307993 | B | 25 June 2014 |
| | | | | CN | 103205407 | A | 17 July 2013 |
| | | | | CN | 103205407 | B | 16 March 2016 |
| | | | | CO | 6341652 | A2 | 21 November 2011 |
| | | | | DK | 3037529 | T3 | 20 May 2019 |
| | | | | EA | 022752 | B1 | 29 February 2016 |
| | | | | EA | 036569 | B1 | 24 November 2020 |
| | | | | EA | 201100908 | A1 | 30 March 2012 |
| | | | | EA | 201500485 | A1 | 30 November 2015 |
| | | | | EP | 2367936 | A1 | 28 September 2011 |
| | | | | EP | 2367936 | B1 | 02 March 2016 |
| | | | | EP | 3037529 | A1 | 29 June 2016 |
| | | | | EP | 3037529 | B1 | 27 March 2019 |
| | | | | ES | 2573462 | T3 | 08 June 2016 |
| | | | | ES | 2724588 | T3 | 12 September 2019 |
| | | | | HK | 1224330 | A1 | 18 August 2017 |
| | | | | HR | P20190766 | T1 | 28 June 2019 |
| | | | | HU | E043591 | T2 | 28 August 2019 |
| | | | | IL | 213150 | A | 31 July 2011 |
| | | | | IL | 213150 | B | 29 May 2017 |
| | | | | IL | 238669 | A | 30 June 2015 |
| | | | | IL | 238669 | B | 31 December 2017 |
| | | | | JP | 2012-511327 | A | 24 May 2012 |
| | | | | JP | 2014-128277 | A | 10 July 2014 |
| | | | | JP | 5670913 | B2 | 18 February 2015 |
| | | | | JP | 5885765 | B2 | 15 March 2016 |

Form PCT/ISA/210 (patent family annex) (July 2022)

**INTERNATIONAL SEARCH REPORT**
Information on patent family members

International application No.

**PCT/KR2022/011586**

| Patent document cited in search report | Publication date (day/month/year) | Patent family member(s) | | | Publication date (day/month/year) |
|---|---|---|---|---|---|
| | | KR | 10-1493644 | B1 | 13 February 2015 |
| | | KR | 10-1546563 | B1 | 28 August 2015 |
| | | KR | 10-2011-0099126 | A | 06 September 2011 |
| | | KR | 10-2014-0021046 | A | 19 February 2014 |
| | | LT | 3037529 | T | 27 May 2019 |
| | | MX | 2011006110 | A | 24 June 2011 |
| | | NZ | 593641 | A | 25 January 2013 |
| | | PL | 3037529 | T3 | 30 August 2019 |
| | | PT | 3037529 | T | 31 May 2019 |
| | | RS | 58728 | B1 | 28 June 2019 |
| | | SG | 172064 | A1 | 28 July 2011 |
| | | SG | 194364 | A1 | 29 November 2013 |
| | | SI | 3037529 | T1 | 28 June 2019 |
| | | TR | 201906944 | T4 | 21 June 2019 |
| | | US | 2012-0213767 | A1 | 23 August 2012 |
| | | US | 8927249 | B2 | 06 January 2015 |
| | | US | 9284543 | B2 | 15 March 2016 |
| | | WO | 2010-077297 | A1 | 08 July 2010 |
| | | ZA | 201104116 | B | 19 December 2018 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS*, 488712-31-8